# EUROPEAN PATENT APPLICATION

(11) **EP 3 127 550 A1**
(43) Date of publication of application: **08.02.2017**
(21) Application number: 15180128.9
(22) Date of filing: 07.08.2015
(51) Int. Cl.: A61K 39/12

(54) **STABILIZING COMPOSITION FOR DRY FORMULATION OF VIRUS**

(71) Applicant: HSC Development GmbH, 3430 Tulln (AT)
(72) Inventor: EGOROV, Andrej, 3430 Tulln (AT)
(74) Representative: Loidl, Manuela Bettina

(57) **Abstract**

The present invention pertains to the field of formulation of viruses, viral particles, viral vaccine suitable for preservation and storage. Specifically, the invention provides a formulation for maintaining a virus in a stable form, comprising said virus and a composition containing a disaccharide, specifically sucrose, carnosine and at least one compound selected from the group of hydroxyethyl starch, monovalent salts, amino acids, high molecular weight proteins and monosodium glutamate monohydrate; methods for stabilizing virus and its use for vaccine formulation.

## Description

The present invention pertains to the field of formulation of virus-based materials, e.g. viruses, viral particles, viral vaccines, suitable for preservation and storage. It also relates to the preparation of such formulation. Specifically, the invention provides a formulation for maintaining a virus in a stable form, comprising virus particles and a composition containing disaccharide, specifically sucrose, carnosine and at least one compound selected from the group of hydroxyethyl starch, monovalent salts, amino acids, high molecular weight proteins and monosodium glutamate monohydrate.

### BACKGROUND OF THE INVENTION

Viruses are important in several therapeutic applications, including, for example, viral therapy and vaccine generation and it is desirable for the viruses to retain their infectivity or immunogenicity. Generally, viruses are very unstable under non-physiological conditions, e.g. ex vivo, and often lose infectivity or immunogenicity after extended periods due to less than optimal formulations or unsuitable storage conditions. Specifically, physical conditions such as high temperature, dry conditions or irradiation usually result in virus inactivation, wherein enveloped viruses generally are more sensitive to those conditions than non-enveloped viruses.

Storage and shipping of virus thus is often problematic since these materials are prone to degradation, especially thermal degradation. This is particularly true in case of vaccines which need to be distributed worldwide and may thus be submitted to different temperatures depending on the countries of distribution or to temperature variation during transport.

Therapeutic activity of virus-based materials requires that structural integrity is maintained during storage and shipping. This structural integrity of a virus-based material is often compromised during the formulation process, thus precluding its therapeutic use. Said therapeutic activity further requires that the viral titer loss, particularly the infectious titer loss, is limited. Developing new methods or formulations to stabilize biologically active materials for industrial applications such as vaccines and to improve storage and shipping abilities of these biological materials is thus a continuous goal of pharmaceutical industry.

One of the proposed solutions has been to maintain the virus within specific temperature ranges, particularly at low temperatures, i.e. below 0°C, more particularly until -30°C and more particularly until -80°C. However, this ultra- low temperature storage is expensive, and creates significant inconvenience for storage, transportation and clinical use. Thus, there also remains a need for long-term storage stable formulations of virus preparations.

Although aqueous vaccine formulation is often preferred due to ease of preparation, costs and handling, unacceptable denaturation and aggregation can be easily induced by numerous stresses to which viral proteins in aqueous solution are very sensitive. Such stresses include ionic strength, heating, agitation, freezing, pH changes, and exposure to interfaces or denaturants (Cleland, J.L., et al., 1993. The development of stable protein formulations: a close look at protein aggregation, deamidation and oxidation. Crit. Rev. Ther. Drug Carrier Syst.10(4), 307-77). Additionally, different degradation processes like hydrolysis or oxidation of viral proteins as well as of excipients can take place in aqueous solution. These degradation processes can be too rapid and do not allow the long storage of vaccines (Goolcharran, C. et al, 2013. Chemical pathways of protein and peptide degradation, Pharmaceutical Formulation Development of Peptides and Proteins, S. Frokjaer and L. Hovgaard, eds., Taylor and Francis, London).

Usually, all biopharmaceuticals are more stable in solid state than in solution. For example, lyophilized formulations can achieve sufficient physical and chemical stability of viral proteins during long-term storage even at elevated temperatures (DeGrazio, F., Flynn, K., 1992. Lyophilization closures for protein based drugs. J. Parenter. Sci. Technol. 46:54). This is related to the reduced mobility of the proteins and the absence of some degradation processes such as hydrolysis.

Therefore one strategy is to preserve virus-based materials in a dried form. Among the available techniques of drying biomaterial, freeze-drying, i.e. lyophilization is a key step for manufacturing vaccines. Freeze-drying leads to dried vaccine which is stable at about 4 to 8 °C and in some cases until about 25°C. Lyophilization has been widely used to improve stability of various viral vaccine and recombinant protein products.

Freeze-drying process involves successive steps of freezing solutions or suspensions of biomaterials, followed by primary and secondary drying steps (Adams, 2007, The principles of freeze-drying, Methods Mol. Biol. 368, 15-38). Basically, this technique is based on sublimation of water at a temperature below 0°C under vacuum without the solution melting. Freezing and drying stages introduce stresses like concentration of salts, precipitation, crystallization, shear stress, pH extremes, or residual moisture remaining through the freeze-drying process that can force the biological material to undergo significant chemical and physical changes and be very damaging to virus-based materials.

Freezing and drying conditions can affect the structural integrity and thereby activity of vaccines. It is thus necessary to have adapted formulations allowing preserving the biologically active material during the drying process, and advantageously further during storage and shipping. Consequently, appropriate stabilizers are necessary for preservation of vaccine during drying and consequent storage.

Cryoprotectants, such as proteins, sugars, polyols, amino acids, dipeptides, inorganic/organic salts can stabilize viral proteins during freeze-thawing cycles and storage at elevated temperatures.

One main problem with the choice of buffering agent is the potential for certain buffer salts to precipitate during freezing which can cause big pH changes. For example, in phosphate based buffers, crystallization of the dibasic form of sodium phosphate during freezing can result in a pH shift to 3 - 4, which will lead to the viral protein denaturation (Van den Berg, L., 1959. The effect of addition of sodium and potassium chloride to the reciprocal system: KH2-PO4-Na2HPO4-H2O on pH and composition during freezing, Arch. Biochem. Biophys. 84:305-315). Thus, phosphate buffers should be avoided. Alternative buffering agents that do not change the pH during freezing could be a zwitterionic organic chemical buffering agent, one of the twenty Good's buffers, HEPES. HEPES dissociation decreases as the temperature decreases, which makes HEPES effective buffering agent for maintaining proteins structure and function at low temperatures (Baicu SC, Taylor MJ., 2002, Acid-base buffering in organ preservation solutions as a function of temperature: new parameters for comparing buffer capacity and efficiency. Cryobiology 45 (1): 33-48). However, phototoxicity of HEPES when exposed to light by the production of hydrogen peroxide is reported. Thus use of HEPES in vaccine formulation is not possible.

Hence, there is a great need for storage-stable active agents, e.g., storage-stable vaccines, with longer shelf life that can maintain efficacy under various robust environmental conditions, e.g., without requiring cold chain compliance. Consequently, there is a need for new formulations allowing stabilization of biological materials, and particularly virus-based materials, allowing industrial applications, storage without affecting biological activity of the product, and more particularly avoiding virus titer loss.

### SHORT DESCRIPTION OF THE INVENTION

The problem is solved by the embodiments of the present invention.

The invention provides a formulation comprising virus, and a composition containing a disaccharide, carnosine and at least one compound selected from the group of hydroxyethyl starch, monovalent salts, amino acids, high molecular weight proteins and monosodium glutamate monohydrate.
According to a specific embodiment, the disaccharide is sucrose.

According to an embodiment, the monovalent salt of the formulation is sodium chloride.

In a further embodiment, the amino acid of the inventive formulation is arginine.

According to an embodiment of the invention, the high molecular weight protein used in the composition is a gelatin hydrolysate or lactalbumin hydrolysate.

In a specific embodiment, the invention provides a formulation containing virus and a composition containing a disaccharide, specifically sucrose, carnosine, hydroxyethyl starch and sodium chloride.

In an alternative embodiment, the invention provides a formulation comprising virus and a composition containing a disaccharide, specifically sucrose, carnosine, hydrolysed gelatin, monosodium glutamate monohydrate and arginine.

According to an embodiment of the invention, the virus can be lyophilized or present in an aqueous solution.

According to a specific embodiment, the virus is influenza virus, specifically live influenza virus, more specifically attenuated influenza virus.

According to a further embodiment of the invention, hydroxyethyl starch has a molecular weight of 100 to 200 kD, specifically about 130 kDa and can be specifically present at a concentration of 1 to 20%, specifically 5 to 10%, more specifically about 6% w/v.

According to a further embodiment, the disaccharide, specifically sucrose, is present at a concentration of 1 to 20%, specifically 2.5 to 10%, more specifically about 5% weight/volume (w/v).

According to a further embodiment, carnosine is present at a concentration of 0.1 to 20%, specifically 0.5 to 10%, more specifically about 1% w/v.

In to a further embodiment, sodium chloride is present at a concentration of 0.01 to 10%, specifically 0.1 to 5%, more specifically about 0.9% w/v.

According to a specific embodiment, the inventive formulation comprises virus and a composition containing 6% w/v hydroxyethyl starch, 5% w/v disaccharide, specifically sucrose, 1% w/v carnosine and 0.9% w/v sodium chloride.

According to an embodiment, a method for stabilizing virus comprising embedding the virus in an aqueous solution containing hydroxyethyl starch, disaccharide, specifically sucrose, carnosine and sodium chloride is provided.

According to a further embodiment, a method for stabilizing virus comprising embedding the virus in an aqueous solution containing disaccharide, specifically sucrose, carnosine, hydroliysed gelatin, monosodium glutamate monohydrate and arginine is provided.

The present invention also provides a method of making a lyophilized viral composition, comprising the steps of:
a) providing a virus and
b) combining the virus with an aqueous solution containing hydroxyethyl starch, disaccharide, specifically sucrose, carnosine and sodium chloride and
c) lyophilizing said formulation to obtain a lyophilisate.

The present invention further provides a method of making a lyophilized viral composition, comprising the steps of:
a) providing a virus and
b) combining the virus with an aqueous solution containing disaccharide, specifically sucrose, carnosine, hydrolysed gelatin, monosodium glutamate monohydrate and arginine and
c) lyophilizing said formulation to obtain a lyophilisate.

Also provided is a method for preserving or stabilizing virus, comprising preparing the inventive formulation and storing said formulation under appropriate conditions.

According to a further embodiment, the formulation according to the invention is used for preparing a medicament.

The invention further provides a stable vaccine suitable for administration to humans produced from a live, attenuated virus, comprising influenza virus stabilized with an aqueous solution containing hydroxyethyl starch, a disaccharide, specifically sucrose, carnosine and sodium chloride.

The invention further provides a stable vaccine suitable for administration to humans produced from a live, attenuated virus, comprising influenza virus stabilized with an aqueous solution containing sucrose, carnosine, hydrolysed gelatin, monosodium glutamate monohydrate and arginine.

The inventive formulation can be used for inducing an immune response in an individual against an infectious disease.

Additionally, a method for treating or preventing a disease condition comprising reconstituting a lyophilized formulation of the invention in water.

### FIGURES

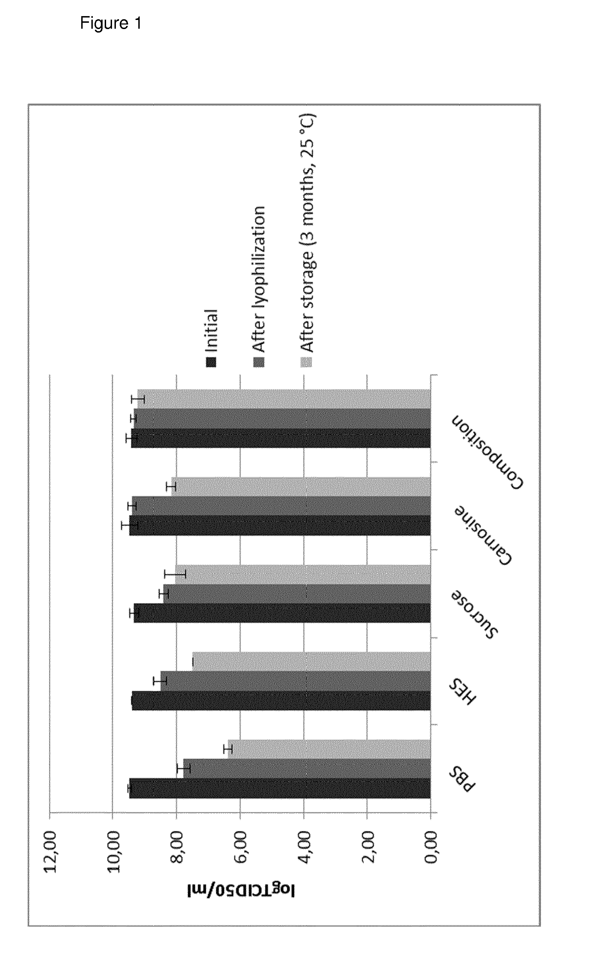

Figure 1: Stability study comprising the logTCID50/ml immediately after resuspension (black bar), after lyophilization process (dark grey bar) and after 3 months storage at room temperature (light grey bar).

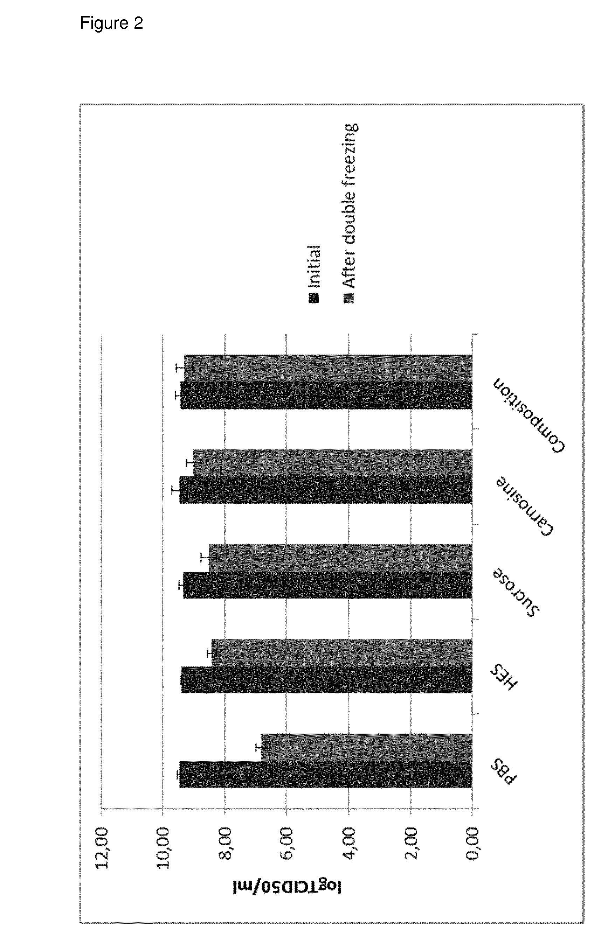

Figure 2: Stability study comprising the logTCID50/ml immediately after resuspension (black bar) and after double freezing (dark grey bar).

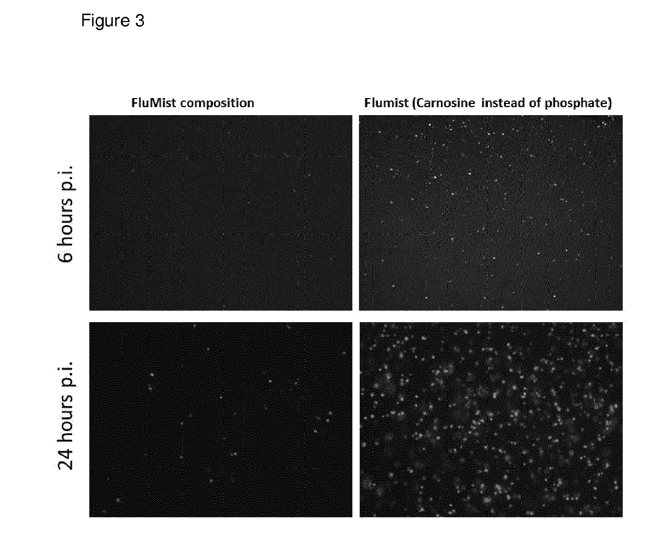

Figure 3: Infection of cells inoculated with virus using the inventive formulation (right lane) and a commercially available composition (left lane) after double freezing.

### DETAILED DESCRIPTION OF THE INVENTION

Described herein are virus formulations for maintaining a virus in a stable form and preparing said formulations. Said formulations can be used to, but are not limited to, retain the infectivity of viruses during prolonged periods of storage and repeated freezing cycles.

The formulation of the invention specifically comprises virus and an aqueous solution containing a disaccharide, specifically sucrose, carnosine and at least one compound selected from the group of hydroxyethyl starch, monovalent salts, amino acids, high molecular weight proteins and monosodium glutamate monohydrate.

The inventors have surprisingly shown that the inventive combination of a disaccharide, specifically sucrose, carnosine and at least one of the above further compounds significantly increased TCID₅₀ for more than 1 log compared to the use of single compounds when used for lyophilization of virus. Even more surprisingly it was shown that the formulation is of significantly increased infectivity even after repeated freezing and thawing, thus providing an excellent virus titer even under harsh conditions.

TCID₅₀/ml is the median tissue culture infective dose; i.e. that amount of a pathogenic agent that will produce pathological change in 50% of cell cultures inoculated.

The virus used according to the invention can be any enveloped or non-enveloped virus. The viruses can be RNA or DNA viruses, specifically enveloped viruses are preferred according to the invention. More specifically the viruses are of the family of orthomyxoviridae, more specifically it is influenza virus. More specifically, it is influenza A, influenza B or influenza C virus, identified by antigenic differences in their nucleoprotein and matrix protein.

Specifically, the virus is a recombinant virus, more specifically it is a reassortant or attenuated virus. Said virus may also contain modifications or deletions in the nonstructural or structural proteins.

The influenza virus can be of any subtype, i.e. H1, H3, H4, H5, H5, H6, H7, H8, H9, H10, H11, H12, H13, H14, H15, H16, H17 etc. More specifically, it can be H1N1, H1N2, H2N2, H3N1, H3N2, H3N8, H5N1, H5N2, H5N3, H5N8, H5N9, H7N1, H7N2, H7N3, H7N4, H7N7, H7N9, H9N2, H10N7.

The viruses for use in the lyophilized formulations described herein can be purified viruses.

As used herein, purified viruses refer to viruses that have been separated from cellular components. Typically, viruses are considered purified when they are at least 70%, at least 75%, at least 80%, at least 85%, at least 90%, at least 95%, at least 99% by dry weight, free from the proteins and other cellular components with which they are naturally associated.

The quantity of viral particles in the present formulation may be determined on the basis of the infectivity of aqueous solutions or spray dried powders produced from formulations comprising the viral particles. Accordingly, a target infectivity level or activity level may be obtained by increasing or decreasing the amount of viral particles in the formulation. Viral particles spray dried in the present formulations typically have similar infectivities after lyophilisation. In some embodiments, viral particles after lyophilisation have at least 70%, at least 75%, at least 80%>, at least 85%, at least 90%, at least 95%, or at least 98% or any other value or range of values therein or thereabove of infectivities or activities prior to lyophilisation.

According to an aspect of the invention, final virus concentration after reconstitution in water can be from 10^{6.5} to 10^{10.5} TCI D50/ml, specifically from 10^{7.5} to 10¹⁰ TCID50/ml, specifically from from 10⁸ to 10¹⁰TCID50/ml, specifically from 10^{8.5} to 10^{9.5} TCID50/ml. "Aqueous solution" according to the invention means purified water, e.g. deonized water or pharmaceutical drade water for injection optionally containing buffer or solvents or any other excipients like surfactants like detergents. Said surfactants may be ionic or non-ionic surfactants. Preferably, the aqueous solution of the invention is substantially free of any excipients. Specifically, the aqueous solution lacks any excipients and additives.

In one aspect, the present invention relates to formulations for spray-drying comprising viral particles. The present formulations are generally aqueous formulations. Typically the formulations are prepared with purified water, e.g., deionized water or pharmaceutical grade water-for-injection (WFI). However, in some embodiments the present formulations may comprise water and one or more solvents which are miscible with water (e.g., ethanol, dimethyl sulfoxide, or any other pharmaceutically acceptable solvent or combination of solvents).

In one aspect, the present invention relates to formulations for spray-drying comprising viral particles. The present formulations are generally aqueous formulations. Typically the formulations are prepared with purified water, e.g., deionized water or pharmaceutical grade water-for-injection (WFI). However, in some embodiments the present formulations may comprise water and one or more solvents which are miscible with water (e.g., ethanol, dimethyl sulfoxide, or any other pharmaceutically acceptable solvent or combination of solvents). Disaccharides are the further compound of the formulation. The disaccharides can be reducing disaccharides, in which one monosaccharide, the reducing sugar, still has a free hemiacetal unit; and non-reducing disaccharides, in which the components bond through an acetal linkage between their anomeric centers and neither monosaccharide has a free hemiacetal unit. Cellobiose and maltose are examples of reducing disaccharides.

According to a preferred embodiment, the disaccharides used in the composition of the invention are selected from sucrose and trehalose which are non-reducing disaccharides.Sucrose can be one compound of the inventive formulation which is known to stabilize proteins during lyophilization (Carpenter, J.F. 1996. Lyophilization of protein pharmaceuticals, Biotechnology and Biopharmaceutical Manufacturing, Processing, and Preservation, Interpharm Press, Buffalo Grove, 199-264). Sucrose protects proteins during both freezing and dehydration and tends to remain amorphous during lyophilization. Additionally, sucrose appears to be a very effective at inhibiting protein unfolding during freeze-drying.

### Sucrose is of the formula

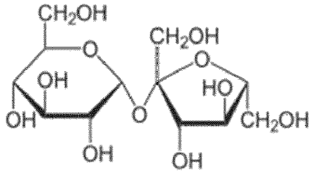

According to an embodiment of the invention, sucrose is present at a concentration of 1 to 20%, specifically 2.5 to 10% w/v. Specifically sucrose is present at a concentration of about 1%, 2%, 3%, 4%,5%, 6%, 7%, 8% or 9% w/v. Specifically it is present at a concentration of between 5% and 7% w/v, more specifically it is present at a concentration of 5% w/v.

Threhalose s a natural alpha-linked disaccharide formed by an α,α-1,1-glucoside bond between two α-glucose units.

### Trehalose is of the formula

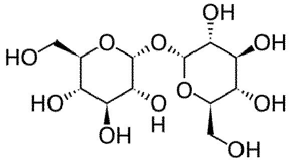

According to an embodiment of the invention, trehalose is present at a concentration of 1 to 20%, specifically 2.5 to 10% w/v. Specifically, trehalose is present at a concentration of about 1%, 2%, 3%, 4%,5%, 6%, 7%, 8% or 9% w/v. Specifically it is present at a concentration of between 5% and 7% w/v, more specifically it is present at a concentration of 5% w/v.

Carnosine is a dipeptide of the amino acids beta-alanine and histidine (ß-alanyl-L-histidine). The IUPAC name is (2*S*)-2-[(3-Amino-1-oxopropyl)amino]-3-(3*H*-imidazol-4-yl)propanoic acid.

### It is of the formula

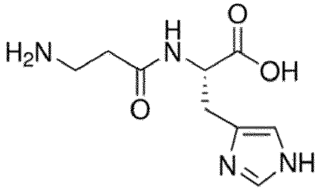

Carnosine is an endogenous dipeptide, abundantly distributed in muscle and nervous tissue in humans. Carnosine has multifunction biological activities including its use as a physiological buffer, wound healing promoter, metal-chelating agent, antioxidant and free radical scavenger and potent anti-inflammatory agent (Decker EA, Livisay SA, Zhou S. A re-evaluation of the antioxidant activity of purified carnosine. Biochemistry (Mosc). 2000 Jul;65(7):766-70)).

According to the invention, carnosine is present at a concentration of 0.1 to 20%, specifically 0.5 to 10% w/v. Specifically, it is present at a concentration of about 0.01%, 0.1%, 0.5%, 1%, 1.5%, 2%, 2.5%, 3%, 3.5%, 4%, 4.5% or 5% w/v. More specifically it is present at a concentration of between 0.5% and 1.5% w/v, specifically 1 % w/v.

Hydroxyethyl starch (HES) which can be comprised in the formulation is generally known as a bulking agent. Lyophilized product with hydroxyethyl starch has strong, porous structure without macroscopic collapse or meltback. A strong, elegant, so called cake structure is obtained. This structure has a high surface area to volume ratio, which aids in the rapid dissolution of product upon addition of water.

HES preparations are mainly distinguished by their molecular weights and additionally by their extent of etherification with hydroxyethyl groups, and by other parameters. The best known representatives of this class of substances are the so-called Hetastarch (HES 450/0.7) and Pentastarch (HES 200/0.5). The latter is the currently most widespread "standard HES". The declared information relating to the molecular weight as well as that relating to the other parameters are averaged quantities, where the molecular weight declaration is based on the weight average (Mw) expressed in Daltons (e.g., for HES 200,000) or in Kilodaltons (kD, e.g., for HES 200). The extent of etherification with hydroxyethyl groups is characterized by the molar substitution MS (e.g. as 0.5 such as in HES 200/0.5; MS, average molar ratio of hydroxyethyl groups to anhydroglucose units) or by the degree of substitution (DS, ratio of mono- or polyhydroxyethylated glucoses to the total anhydroglucose units). According to their molecular weights, the HES solutions in clinical use are classified into high-molecular weight (450 kD), medium-molecular weight (200-250 kD) and low-molecular weight (70-130 kD) preparations.

All known starches are suitable for the preparation of HES used according to the invention, mainly these starches are native or partially hydrolyzed starches, preferably cereal or potato starches, especially those having a high content of amylopectin.

### Hydroxyethyl starch has the general formula

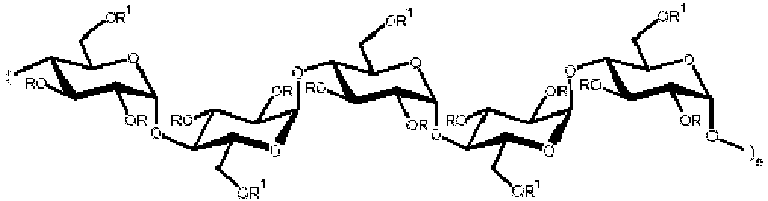

R = -H,-CH₂CH₂OH
R₁ = -H,-CH₂CH₂OH or glucose units

In a preferred embodiment of the invention, hydroxyethyl starch, poly(O-2-hydroxyethyl) starch, specific molar substitution 0.38-0.45, can be medium or low molecular weight HES. Low molecular weight starch is preferred. Specifically HES has a mean molecular weight of 100 to 200 kDa, specifically about 120-140 kDa, more specifically about 130 kDa. More specifically it is hydroxyethyl starch 130/0.4.

As an example, Voluven ®, Hydroxyethyl starch (HES) 130/0.4 in 0.9% sodium chloride isotonic solution known for intravenous administration can be used as starting solution comprising HES and sodium chloride.

According to the invention, HES can be present at a concentration of 1 to 20%, specifically 5 to 10%. More specifically, HES is present at a concentration of about 1%, 2%, 3%, 4%, 5%, 6%, 7%, 8%, 9%, 10% w/v. More specifically, HES is at a concentration of 6% w/v.

In order to adjust tonicity, the present formulation may contain a monovalent cationic salt, such as, for example, sodium (Na⁺), lithium (Li⁺), potassium (K⁺), and ammonium (NH₄⁺) containing salts. Specifically, the formulation contains NaCl.

According to the invention, the salt, e.g. NaCl is present at a concentration of 0.01 to 10%, specifically 0.1 to 5% w/v. Specifically NaCl is present at a concentration of about 0.1%, 0.2%, 0.3%, 0.4%, 0.5%, 0.6%, 0.7%, 0.8%, 0.9%, 1%, 1.5%, 2%, 2.5%, 3%, 3.5%, 4%, 4.5%, 5% w/v. More specifically NaCl is contained at a concentration of 0.9% w/v.

The inventive formulation may also contain one or more amino acids or salts thereof mixtures thereof. Specifically the amino acids are selected from the group of charged amino acids, more specifically it is arginine.

The formulation of the invention can also comprise a salt of glutamic acid, i.e. monosodium glutamate monohydrate or sodium glutamate.

According to the invention, the amino acid or its salt is present at a concentration between 0.1% and 5% w/v, specifically between 1% and 2% w/v, more specifically at a concentration of about 1.2%.

High molecular weight proteins which can be contained in the inventive formulation can be any protein hydrolysates from any source which are applicable for administration to the individuals body. Specifically said hydrolysates are gelatin hydrolysate or lactalbumin hydrolysate. According to the invention, the high molecular weight proteins are present at a concentration between 0.01 and 15% w/v, specifically between 0.1% and 10%, more specifically between 0.4 and 0.8%, more specifically at a concentration of about 0.5% w/v.

According to the invention, the term "about" encompasses the explicitly recited values as well as small deviations therefrom. Accordingly, a deviation from a recited value for 10%, preferably 5%, preferably 1% is encompassed by the term "about".

In a specific embodiment the formulation comprises virus and an aqueous composition containing 5-10% w/v hydroxyethyl starch, 2.5-7.5 % w/v sucrose, 0.1 to 2% w/v carnosine and 0.5 - 1% w/v sodium chloride.

In a specific embodiment, the formulation comprises virus and an aqueous composition containing 6% w/v hydroxyethyl starch, 5% w/v sucrose, 1% w/v carnosine and 0.9% w/v sodium chloride.

In a further specific embodiment, the formulation consists of virus and a composition consisting of water, 6% w/v hydroxyethyl starch, 5% w/v disaccharide, specifically sucrose, 1% w/v carnosine and 0.9% w/v sodium chloride.

Specifically, the inventive formulation is substantially free of other excipients or additives than virus, HES, disaccharide, specifically sucrose, carnosine and sodium chloride.

In a specific embodiment the formulation comprises virus and an aqueous composition containing 5-8% w/v disaccharide, specifically sucrose, 0.1 to 2% w/v carnosine, 2.5 - 7.5% hydrolysed porcine gelatin, 0.5 - 1.5% w/v monosodium glutamate monohydrate and 1.0 - 2% w/v arginine.

In a specific embodiment the formulation comprises virus and an aqueous composition containing 6.8% w/v disaccharide, specifically sucrose, 1% w/v carnosine, 0.5% hydrolysed porcine gelatin, 1% w/v monosodium glutamate monohydrate and 1.2% w/v arginine.

In a further specific embodiment, the formulation consists of virus and a composition consisting of water, 6.8% w/v disaccharide, specifically sucrose, 1% w/v carnosine, 0.5% hydrolysed porcine gelatin, 1 % w/v monosodium glutamate monohydrate and 1.2% w/v arginine.

More particularly, the formulation of the invention is suitable for drying processes, more particularly freeze-drying processes. The invention further concerns the obtained dried product, and more particularly the obtained freeze-dried product as well as the product redissolved in water.

The aqueous formulations and dried products according to the present invention are useful as vaccine for preventing and/or treating disorders, specifically of infectious diseases.

The term "lyophilized preparation", as used according to the invention, refers to a preparation in which the liquid part has been removed or reduced. Suitable methods for drying a virus preparation include, but are not limited thereto, lyophilization (freeze-drying), spray-drying, freeze- spray drying, air drying or foam drying. The methods are known in the art.

The aqueous part is considered to have been reduced if the liquid is reduced to less than 20% of the volume, specifically than 10%, specifically less than 5%, more specifically less than 3%, specifically less than 2 %, more specifically less than 1 % of the volume.

According to a specific embodiment, the liquid is reduced to 0.5% or less.

Specifically, the virus formulations are stable at about ambient temperature for a period of time, e.g. at least one day. Specifically, the viral formulations are stable at a temperature of about 4° C or lower for at least three months, e.g., at least six months, at least twelve months, at least eighteen months, or any duration longer than three months. Specifically, the virus formulation is suitable for reconstitution before administration. Reconstituted virus formulations can be further diluted to achieve a preferred dose for administration.

A method for stabilizing virus is also provided, wherein the virus is embedded in an aqueous solution containing hydroxyethyl starch, disaccharide, specifically sucrose, carnosine and sodium chloride.

A method for stabilizing virus is also provided, wherein the virus is embedded in an aqueous solution containing disaccharide, specifically sucrose, carnosine, hydrolysed gelatin, sodium glutamate and arginine.

The term "stabilizing" according to the invention means that infectivity of the virus is preserved.

The term "infectivity", as used herein, relates to the ability of a virus to establish an infection in a host or host cell. More specifically, infectivity is the capacity for horizontal transmission of the virus, i.e. its spread between hosts rather than from parent to child. Means for determining whether a virus has maintained its infectivity are well known in the art and include, without being limiting, testing a cell culture obtained from a host with regard to replication of the virus, PCR analysis, animal models as well as electron-optical analysis.

A virus is considered to have maintained its infectivity in accordance with the present invention when the amount of infectivity is at least 30% of the infectivity of said organism prior to embedding it in the inventive solution. More preferably, the amount of infectivity is at least 50%, more preferably at least 70%, such as at least 95% and most preferably at least 98% of the infectivity of said organism prior to embedding it in the inventive solution containing HES, sucrose, carnosine and NaCl.

In accordance with the present invention, viruses embedded in the inventive aqueous solution not only survive for a prolonged period of time, but also maintain their infectious capabilities and their ability to reproduce.

The invention provides a method of making a lyophilised viral formulation, comprising the steps of providing a virus, combining the virus with an aqueous solution containing hydroxyethyl starch, disaccharide, , carnosine and sodium chloride and lyophilizing said formulation to obtain a lyophilisate.

The invention alternatively provides a method of making a lyophilised viral formulation, comprising the steps of providing a virus, combining the virus with an aqueous solution containing disaccharide, specifically sucrose, carnosine, hydrolysed gelatin, sodium glutamate and arginine and lyophilizing said formulation to obtain a lyophilisate.

Lyophilization or freeze-drying is well known in the art and includes the steps of freezing the organisms and subsequently reducing the surrounding pressure while adding sufficient heat to allow the frozen water in the material to sublime directly from the solid phase to the gas phase. Preferably, the lyophilized preparation is then sealed to prevent the re-absorption of moisture.

Spray-drying is also well known in the art and is a method to convert a solution, suspension or emulsion into a solid powder in one single process step. Generally, a concentrate of the liquid product is broken into small droplets. These droplets meet a stream of hot air wherein moisture is reduced rapidly while still suspended in the drying air. The dry powder is separated from the moist air by centrifugation.

Spray-freeze-drying is a method that combines processing steps common to freeze-drying and spray-drying. The virus provided in the solution according to the invention is nebulized into a cryogenic medium, for example liquid nitrogen which produces shock-frozen droplets which are dried in a lyophiliser.

According to the invention, the viruses can be stabilized in the inventive aqueous solution and can be subsequently subjected to a drying step. The reduced water content inhibits the action of e.g. enzymes that could degrade the virus and therefore provides additional protection. Furthermore, surface antigens necessary for host cell binding present on the surface of the virus are protected during the drying step, thus maintaining the antigenicity of the viruses as well as the interaction with host cells. After reconstitution, the viruses may be employed as live virus vaccines or as attenuated or killed virus vaccines via the presentation of surface antigens.

The formulation specifically may have a pH in the range of 7.5 and 8.5, specifically about 8.0.

Under specific circumstances the virus may be inactivated after embedding it in the inventive solution.

According to the invention, the composition comprising HES, disaccharide, specifically sucrose, carnosine and NaCl can also be added to a culture of cells infected with virus.

As summarized above, the present invention provides a method for preserving or stabilizing virus, comprising preparing the inventive formulation and storing said formulation.

According to the invention, the formulation containing virus, HES, disaccharide, specifically sucrose, carnosine and NaCl is useful for preparing a medicament. Said medicament can be a vaccine formulation for active immunization of individuals against the respective virus.

Specifically a stable vaccine suitable for administration to humans produced from a live, attenuated virus, comprising influenza virus stabilized with an aqueous solution containing hydroxyethyl starch, disaccharide, specifically sucrose, carnosine and sodium chloride. Said vaccine can be used for inducing an immune response in an individual against an infectious disease.

The respective vaccine may contain virus of one type but may also contain a combination of different types of virus.

The lyophilized viral formulation optionally reconstituted in water, is administered in a manner so that the virus can ultimately contact the target cells, for example, systemically. The route by which the viral formulation is administered depends on the location as well as the type of the target cells. A wide variety of administration routes can be employed. For vaccines, the virus can be administered systemically, orally, sublingually, intranasally, intradermally, or subcutaneously, so as to target antigen presenting cells, so as to elicit an immune response.

Further comprised in the present invention is a method for treating or preventing a disease condition comprising reconstituting a lyophilized formulation of the invention in water.

In addition, the invention provides containers and kits containing the inventive formulations

The invention further encompasses following items:
1. Formulation or composition for maintaining a virus in a stable form, comprising said virus and a composition containing disaccharide, carnosine and at least one compound selected from the group of hydroxyethyl starch, monovalent salts, amino acids, high molecular weight proteins and monosodium glutamate monohydrate.
2. The formulation according to item 1, wherein the monovalent salt is sodium chloride.
3. The formulation according to claim 1 or 2, wherein the disaccharide is sucrose or trehalose, specifically it is sucrose.
4. The formulation according to any one of items 1 to 3, wherein the amino acid is arginine.
5. The formulation according to any one of items 1 to 4, wherein the high molecular weight protein is a gelatin hydrolysate or lactalbumin hydrolysate.
6. The formulation according to any one of items 1 to 5, comprising virus and a composition containing disaccharide, carnosine, hydroxyethyl starch and sodium chloride.
7. The formulation according to any one of items 1 to 5, comprising virus and a composition containing disaccharide, carnosine, hydrolysed gelatin, monosodium glutamate monohydrate and arginine.
8. The formulation according to any one of items 1 to 7, wherein the virus is lyophilized or in the form of aqueous solution.
9. The formulation according to any one of items 1 to 8, wherein said virus is influenza virus, specifically attenuated influenza virus.
10. The formulation according to any one of items 1 to 9, wherein hydroxyethyl starch has a molecular weight of 100 to 200kD, specifically about 130kDa.
11. The formulation according to any one of items 1 to 10, wherein hydroxyethyl starch is present at a concentration of 1 to 20%, specifically 5 to 10%, more specifically about 6% w/v.
12. The formulation according to any one of items 1 to 111, wherein sucrose is present at a concentration of 1 to 20%, specifically 2.5 to 10%, more specifically about 5% w/v.
13. The formulation according to any one of items 1 to 12, wherein carnosine is present at a concentration of 0.1 to 20%, specifically 0.5 to 10%, more specifically about 1 % w/v.
14. The formulation according to any one of items 1 to 13, wherein sodium chloride is present at a concentration of 0.01 to 10%, specifically 0.1 to 5%, more specifically about 0.9% w/v.
15. The formulation according to any one of items 1 to 14, wherein the amino acid or its salt is present at a concentration of 0.1 to 5%, specifically at 1 to 2%, specifically about 1.2% w/v.
16. The formulation according to any one of items 1 to 14, wherein the high molecular weight protein is present at a concentration of 0.01 to 15%, specifically 0.1 to 10%, more specifically 0.4 to 0.8%, more specifically 5% w/v.
17. The formulation comprising virus and a composition containing 6% w/v hydroxyethyl starch, 5% w/v disaccharide, 1 % w/v carnosine and 0.9% w/v sodium chloride.
18. The formulation comprising virus and a composition containing 5% w/v disaccharide, 1% w/v carnosine, 0.5% hydrolysed gelatin, 0.1% sodium glutamate and 1.2% arginine.
19. Method for stabilizing virus comprising embedding the virus in an aqueous solution containing hydroxyethyl starch, disaccharide, carnosine and sodium chloride.
20. Method for stabilizing virus comprising embedding the virus in an aqueous solution containing disaccharide, carnosine, hydrolysed gelatin, sodium glutamate and arginine.
21. Method of making a lyophilised viral formulation, comprising the steps of:
   a. providing a virus and
   b. combining the virus with an aqueous solution containing hydroxyethyl starch, disaccharide, carnosine and sodium chloride and
   c. lyophilizing said formulation to obtain a lyophilisate.
22. Method of making a lyophilised viral formulation, comprising the steps of:
   a. providing a virus and
   b. combining the virus with an aqueous solution containing disaccharide, carnosine, hydrolysed gelatin, sodium glutamate and arginine and
   c. lyophilizing said formulation to obtain a lyophilisate.
23. Method for preserving or stabilizing virus, comprising preparing a formulation according to any one of items 1 to 18 and storing said formulation.
24. The formulation according to any one of items 1 to 18 for preparing a medicament.
25. A stable vaccine suitable for administration to humans produced from a live, attenuated virus, comprising influenza virus stabilized with an aqueous solution containing hydroxyethyl starch, disaccharide, carnosine and sodium chloride.
26. A stable vaccine suitable for administration to humans produced from a live, attenuated virus, comprising influenza virus stabilized with an aqueous solution containing disaccharide, carnosine hydrolysed gelatin, sodium glutamate and arginine.
27. The formulation according to any one of items 1 to 18 for inducing an immune response in an individual against an infectious disease.
28. A method for treating or preventing a disease condition comprising reconstituting in water a lyophilized formulation according to any one of items 1 to 18.

The examples described herein are illustrative of the present invention and are not intended to be limitations thereon. Different embodiments of the present invention have been described according to the present invention. Many modifications and variations may be made to the techniques described and illustrated herein without departing from the spirit and scope of the invention. Accordingly, it should be understood that the examples are illustrative only and are not limiting upon the scope of the invention.

### EXAMPLES

### Example 1

### Stabilizing composition of dry formulation for life influenza vaccines.

### Test composition.

1. HES, Hydroxyethyl starch (Poly(O-2-hydroxyethyl)starch), molar substitution 0,38-0,45, mean molecular weight 130 kDa (HES 130/0,4), 6 % w/v.
2. Sucrose, 5% w/v.
3. Carnosine, 1 % w/v.
4. Sodium Chloride, 0,9% w/v.

### Samples preparation.

The test virus A/Puerto Rico/8/1934 H1 N1 (pr8) was obtained by propagation in 10 days old pathogen-free embryonated chicken eggs. The virus was concentrated and purified from external proteins by precipitation with polyethylene glycol 6000 followed by centrifugation (130000 g) through 30 % sucrose gradient. The final viral pellets were equally resuspended in following solutions:
1. PBS (Phosphate-buffered saline)
2. HES, 6 % w/v + Sodium Chloride, 0,9% w/v
3. Sucrose, 5% w/v + Sodium Chloride, 0,9% w/v
4. Carnosine, 1 % w/v + Sodium Chloride, 0,9% w/v
5. Test composition

### Stability study.

All samples were lyophilized and placed at + 25 °C.

Immediately after resuspension, after lyophilisation process and after storage at + 25 °C for 3 months samples were assessed on their infectious titers (TCID 50).

Additionally, in order to assess all compositions on their ability to prevent virus damage during freezing, all samples were twice freezed/thawed (freezing: 0.5 hour at - 70 °C, thawing: 0.5 hour at +25 °C) and their infectious titers determined.

**Table 1**

| **Infectious titer TCID50**/**ml** | | | | | |
|---|---|---|---|---|---|
| **Initial (after purification)** | | | | | |
| | 1st | 2nd | 3rd | Mv | SD |
| **PBS** | 9,50 | 9,50 | 9,40 | **9,47** | 0,06 |
| **HES** | 9,40 | 9,40 | 9,40 | **9,40** | 0,00 |
| **Sucrose** | 9,25 | 9,50 | 9,25 | **9,30** | 0,14 |
| **Carnosine** | 9,75 | 9,25 | 9,40 | **9,47** | 0,26 |
| **Composition** | 9,40 | 9,25 | 9,60 | **9,42** | 0,18 |

**Table 2**

| **Infectious titer TCID50/ml** | | | | | |
|---|---|---|---|---|---|
| **After lyophilization** | | | | | |
| | 1st | 2nd | 3rd | Mv | SD |
| **PBS** | 8,00 | 7,60 | 7,75 | **7,78** | 0,20 |
| **HES** | 8,40 | 8,40 | 8,75 | **8,52** | 0,20 |
| **Sucrose** | 8,50 | 8,25 | 8,50 | **8,42** | 0,14 |
| **Carnosine** | 9,40 | 9,25 | 9,50 | **9,38** | 0,13 |
| **Composition** | 9,40 | 9,40 | 9,25 | **9,35** | 0,09 |

**Table 3**

| **Infectious titer TCID50/ml** | | | | | |
|---|---|---|---|---|---|
| **After storage, 3 month 25°C** | | | | | |
| | 1st | 2nd | 3rd | Mv | SD |
| **PBS** | 6,50 | 6,40 | 6,24 | **6,38** | 0,13 |
| **HES** | 7,50 | 7,50 | 7,50 | **7,50** | 0,00 |
| **Sucrose** | 8,00 | 7,75 | 8,40 | **8,05** | 0,33 |
| **Carnosine** | 8,00 | 8,25 | 8,25 | **8,17** | 0,14 |
| **Composition** | 9,25 | 9,00 | 9,40 | **9,22** | 0,20 |

**Table 4**

| **Infectious titer TCID50/ml** | | | | | |
|---|---|---|---|---|---|
| **After double freezing** | | | | | |
| | 1st | 2nd | 3rd | Mv | SD |
| **PBS** | 7,00 | 6,75 | 6,75 | **6,83** | 0,14 |
| **HES** | 8,50 | 8,25 | 8,50 | **8,42** | 0,14 |
| **Sucrose** | 8,50 | 8,75 | 8,25 | **8,50** | 0,25 |
| **Carnosine** | 9,00 | 9,25 | 8,75 | **9,00** | 0,25 |
| **Composition** | 9,40 | 9,50 | 9,00 | **9,30** | 0,26 |

### Example 2:

### 2 compositions were tested:

### 1. Stabilizing composition of commercially available live attenuated influenza vaccine FluMist:

- Sucrose 68,4 g/l
- Hydrolysed porcine gelatin (Gelatin from porcine skin, Type A, Sigma G6144-100G) 5 g/l
- Monosodium glutamate monohydrate 1,04 g/l
- Arginine 12,1 g/l
- K₂HPO₄ 11,3 g/l
- KH₂PO₄ 4,8 g/l

### 2. Composition containing attenuated influenza virus containing carnosine

- Sucrose 68,4 g/l
- Hydrolysed porcine gelatin (Gelatin from porcine skin, Type A, Sigma G6144-100G) 5 g/l
- Monosodium glutamate monohydrate 1,04 g/l
- Arginine 12,1 g/l
- Carnosine 10,0 g/l

**Experimental design:** Influenza virus A/Puerto Rico/8/1934 H1 N1 (pr8 GFP) expressing Green Fluorescent Protein reporter (GFP) from the NS1 reading frame was used for infection of Vero cells. Cells were inoculated at MOI=3 without presence of trypsin with pr8 GFP virus suspended in alternative compositions 1 and 2. After 0,5 hour inoculation, tested solutions were exchanged with cells maintains OptiPro medium. Pictures were taken at 6 and 24 hours of incubation at 37 °C. The number of infected cells was estimated according to their fluorescence. Figure xx shows enhanced infection of cells inoculated with composition 2, containing carnosine.

## Claims

1. Formulation for maintaining a virus in a stable form, comprising said virus and a composition containing a disaccharide, specifically sucrose, carnosine and at least one compound selected from the group of hydroxyethyl starch, monovalent salts, amino acids, high molecular weight proteins and monosodium glutamate monohydrate.

2. The formulation according to claim 1, wherein the monovalent salt is sodium chloride.

3. The formulation according to claim 1 or 2, wherein the amino acid is arginine.

4. The formulation according to any one of claims 1 to 3, wherein the high molecular weight protein is a gelatin hydrolysate or lactalbumin hydrolysate.

5. The formulation according to any one of claims 1 to 4, comprising virus and a composition containing disaccharide, specifically sucrose, carnosine, hydroxyethyl starch and sodium chloride.

6. The formulation according to any one of claims 1 to 4, comprising virus and a composition containing disaccharide, specifically sucrose, carnosine, hydrolysed gelatin, monosodium glutamate monohydrate and arginine.

7. The formulation according to any one of claims 1 to 6, wherein the virus is lyophilized or in the form of an aqueous solution, specifically said virus is influenza virus, specifically attenuated influenza virus.

8. The formulation according to any one of claims 1 to 7, wherein hydroxyethyl starch has a molecular weight of 100 to 200kD, specifically about 130kDa.

9. The formulation according to any one of claims 1 to 8, wherein hydroxyethyl starch is present at a concentration of 1 to 20%, specifically 5 to 10%, more specifically about 6% w/v.

10. The formulation according to any one of claims 1 to 9, wherein sucrose is present at a concentration of 1 to 20%, specifically 2.5 to 10%, more specifically about 5% w/v.

11. The formulation according to any one of claims 1 to 10, wherein carnosine is present at a concentration of 0.1 to 20%, specifically 0.5 to 10%, more specifically about 1 % w/v.

12. The formulation according to any one of claims 1 to 11, wherein sodium chloride is present at a concentration of 0.01 to 10%, specifically 0.1 to 5%, more specifically about 0.9% w/v.

13. The formulation according to any one of claims 1 to 12, wherein the amino acid or its salt is present at a concentration of 0.1 to 5%, specifically of 1 to 2%, specifically about 1.2% w/v.

14. The formulation according to any one of claims 1 to 13, wherein the high molecular weight protein is present at a concentration of 0.01 to 15%, specifically 0.1 to 10%, more specifically 0.4 to 0.8%, more specifically 5% w/v.

15. Method for stabilizing virus comprising embedding the virus in an aqueous solution containing hydroxyethyl starch, a disaccharide, specifically sucrose, carnosine and sodium chloride.
